# EUROPEAN PATENT APPLICATION

(11) **EP 4 699 639 A1**
(43) Date of publication of application: **25.02.2026**
(21) Application number: 24807422.1
(22) Date of filing: 08.05.2024
(51) Int. Cl.: A61M 39/10, A61M 5/142

(54) **PORT CONNECTOR AND DRUG INJECTION DEVICE COMPRISING SAME**

(30) Priority: 16.05.2023 KR 20230063009
(71) Applicant: Caremedi Co., Ltd., Seoul 07207 (KR); Sogang University Research & Business Development Foundation, Seoul 04107 (KR)
(72) Inventor: SHIN, Woonsup, Seoul 04065 (KR); ZHU, Enhua, Seoul 04109 (KR); LEE, Junghyun, Seoul 03916 (KR)
(74) Representative: Manitz Finsterwald Patent- und Rechtsanwaltspartnerschaft mbB
(86) International application number: PCT/KR2024/006146
(87) International publication number: WO 2024/237546

(57) **Abstract**

A drug injection device according to the present disclosure includes: a housing which includes a lid and a contact surface and in which a mounting space is formed between the lid and the contact surface; a drug reservoir which is disposed in the mounting space and in which a drug is stored; a pump assembly which electrochemically generates positive and negative pressures to suck and discharge the drug from the drug reservoir; a port connector which is disposed between the drug reservoir and the pump assembly and coupled to the drug reservoir and the pump assembly, and provides a first flow path through which the drug sucked from the drug reservoir to the pump assembly moves and a second flow path through which the drug discharged from the pump assembly moves; and an insertion tube coupled to the port connector and configured to inject the drug into a user.

## Description

### TECHNICAL FIELD

The technical field of the present disclosure relates to a port connector and a drug injection device including the same.

### BACKGROUND

Drugs may be injected into the body in various ways, such as oral, subcutaneous, and intravenous administration, depending on the type of drug and the purpose and method of treatment. A drug injector using a drug pump may automatically inject a drug into the body at a desired speed and dose at a required time. Therefore, a drug injector using a drug pump may be used not only in hospitals or in patients' daily living environments, but also in various forms.

An insulin pump, which is commonly called an insulin injector, is a medical device that supplies insulin into the body from the outside at a predetermined time to accurately control the blood sugar level, similar to the pancreas, for a diabetic patient who does not secrete insulin or secretes only a small amount of insulin.

Such an insulin pump is used by insulin-dependent diabetic patients, and may continuously inject a drug for 24 hours in a state of being attached to the patient. In this way, the insulin injector needs to periodically inject a drug into a diabetic patient for a long period of time. Therefore, technological development is actively underway to miniaturize and automate insulin injectors for the convenience of users.

As insulin injectors become more miniaturized and automated, their internal structure is likely to change. However, whenever a specific component is changed, it is difficult to replace the entire assembly coupled to the component from a manufacturing standpoint.

Accordingly, there is a need for a device capable of flexibly accommodating changes in specific components.

### DISCLOSURE OF THE INVENTION

### PROBLEMS TO BE SOLVED BY THE INVENTION

In view of the foregoing, the present disclosure is conceived to provide a port connector to be coupled to a drug reservoir and a pump assembly.

Also, the present disclosure is conceived to provide a drug injection device equipped with a port connector to be coupled to a drug reservoir, a pump assembly, and an injection assembly.

The problems to be solved by the present disclosure are not limited to the above-described problems. There may be other problems to be solved by the present disclosure.

### MEANS FOR SOLVING THE PROBLEMS

As a means for solving the above-described problems, a port connector according to the present disclosure includes: a main body coupled between a drug reservoir and a pump assembly; and first and second flow paths which are formed inside the main body and through which a drug moves. The first flow path is a flow path through which the drug moves from the drug reservoir toward the pump assembly, and the second flow path is a flow path through which the drug discharged from the pump assembly moves.

Further, a drug injection device according to an embodiment of the present disclosure includes: a housing which includes a lid and a contact surface and in which a mounting space is formed between the lid and the contact surface; a drug reservoir which is disposed in the mounting space and in which a drug is stored; a pump assembly which electrochemically generates positive and negative pressures to suck and discharge the drug from the drug reservoir; a port connector which is disposed between the drug reservoir and the pump assembly and coupled to the drug reservoir and the pump assembly, and provides a first flow path through which the drug sucked from the drug reservoir to the pump assembly moves and a second flow path through which the drug discharged from the pump assembly moves; and an insertion tube coupled to the port connector and configured to inject the drug into a user.

### EFFECTS OF THE INVENTION

According to the means for solving the problems of the present disclosure, the versatility of an electroosmotic pump can be enhanced by a port connector that serves as a medium for delivering a drug between the electroosmotic pump and other components.

### BRIEF DESCRIPTION OF THE DRAWINGS

**FIG. 1** is a front perspective view of a drug injection device according to an embodiment of the present disclosure.
**FIG. 2** is a rear perspective view of the drug injection device according to an embodiment of the present disclosure.
**FIG. 3** is an internal plan view of a housing illustrated in **FIG. 1****.**
**FIG. 4** is a plan view of a drug reservoir illustrated in **FIG. 3****.**
**FIG. 5** is a plan view of a pump assembly illustrated in **FIG. 3****.**
**FIG. 6** is a block diagram schematically illustrating a configuration of the pump assembly illustrated in **FIG. 5****.**
**FIG. 7** is a block diagram schematically illustrating a configuration of a driver illustrated in **FIG. 6****.**
**FIG. 8** is an example diagram schematically illustrating an operation of the driver illustrated in **FIG. 7****.**
**FIG. 9** is a perspective view illustrating a coupling structure of the drug reservoir, the pump assembly, and the port connector illustrated in **FIG. 3****.**
**FIG. 10** is a front perspective view of the port connector illustrated in **FIG. 3****.**
**FIG. 11** is a cross-sectional view taken along a line A-A' of **FIG. 10****.**
**FIG. 12** is a rear perspective view of the port connector illustrated in **FIG. 3****.**
**FIG. 13** is a cross-sectional view taken along a line B-B' of **FIG. 12****.**

### BEST MODE FOR CARRYING OUT THE INVENTION

Hereafter, embodiments will be described in detail with reference to the accompanying drawings. However, it is to be noted that the present disclosure is not limited to the embodiments but can be embodied in various other ways. Also, the accompanying drawings are provided only for a better understanding of the embodiments disclosed in the present disclosure and are not intended to limit technical ideas disclosed in the present disclosure. In the drawings, parts irrelevant to the description are omitted for the simplicity of explanation, and the size, form and shape of each component illustrated in the drawings can be modified in various ways. Like reference numerals denote like parts throughout the whole document.

Suffixes "assembly" and "unit" used for components disclosed in the following description are merely intended for easy description of the specification, and the suffixes themselves do not give any special meaning or function. Further, in the following description of the present disclosure, a detailed explanation of known related technologies may be omitted to avoid unnecessarily obscuring the subject matter of the present disclosure.

Throughout the whole document, the term "connected to (contacted with or coupled to)" may be used to designate a connection or coupling of one element to another element and includes both an element being "directly connected to (contacted with or coupled to)" another element and an element being "indirectly connected to (contacted with or coupled to)" another element via another element. Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Further, in describing components of the present disclosure, ordinal numbers such as first, second, etc. can be used only to differentiate the components from each other, but do not limit the sequence or relationship of the components. For example, a first component of the present disclosure may also be referred to as a second component and vice versa.

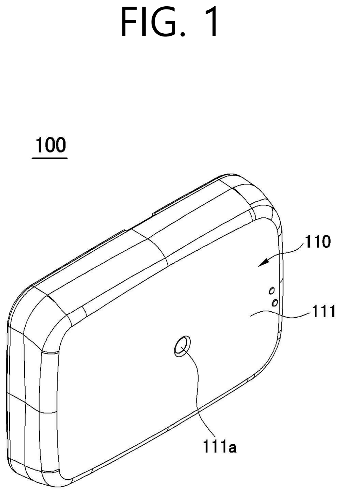

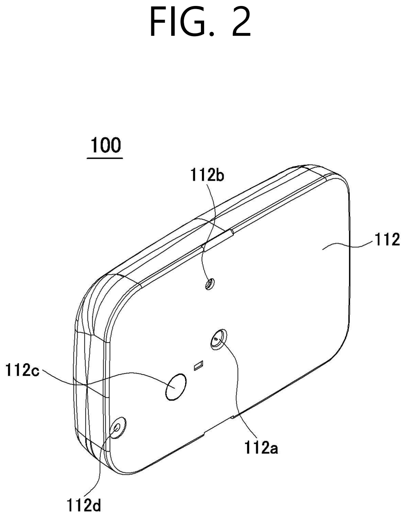

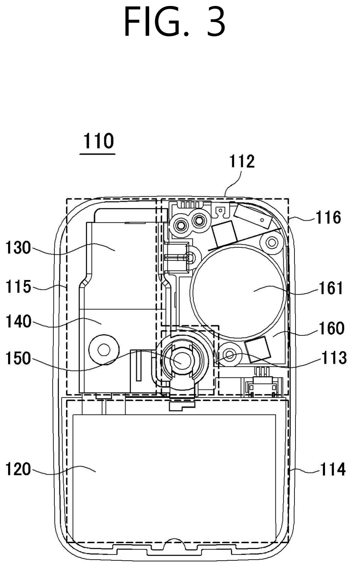

**FIG. 1** is a front perspective view of a drug injection device according to an embodiment of the present disclosure, **FIG. 2** is a rear perspective view of the drug injection device according to an embodiment of the present disclosure, and **FIG. 3** is an internal plan view of a housing illustrated in **FIG. 1****.**

Referring to **FIG. 1** to **FIG. 3****,** a patch-type drug injection device 100 according to an embodiment of the present disclosure includes a housing 110 and components disposed within the housing 110.

The housing 110 includes a lid 111 and a contact surface 112, and a mounting space is formed between the lid 111 and the contact surface 112. The lid 111 has rounded corners, and, thus, the patch-type drug injection device 100 can remain continuously attached to an injection target without a foreign body sensation. The contact surface 112 is configured to be attached to the injection target, and a discharge port 112a is formed therein to draw an insertion tube outward.

The mounting space is divided into a central region 113 and first to third regions 114, 115 and 116. The first to third regions 114, 115 and 116 are formed to surround the central region 113. In particular, the mounting space within the housing 110 is divided into the central region 113 and the first to third regions 114, 115 and 116, which extend in a direction perpendicular to an insertion direction of the insertion tube. Major components are coupled to each of the regions. In a conventional device, a PCB circuit unit is first disposed over the entire or most of the plane of the device, and a drug reservoir or pump is disposed above the circuit unit, which causes the overall thickness of the device to increase. However, in the patch-type drug injection device 100 according to the present disclosure, the thickness of the device 100 is reduced by minimizing the overlapping areas between components.

The components include a drug reservoir 120, a pump assembly 130, a port connector 140, an injection assembly 150, and a circuit unit 160, and may be disposed in the central region 113 and the first to third regions 114, 115 and 116 described above. For example, the drug reservoir 120 may be disposed in the first region 114, the pump assembly 130 and the port connector 140 may be disposed in the second region 115, and the circuit unit 160 may be disposed in the third region 116. Further, the injection assembly 150 may be disposed in the central region 113, and the drug reservoir 120, the pump assembly 130, the port connector 140, and the circuit unit 160 may be arranged around the injection assembly 150.

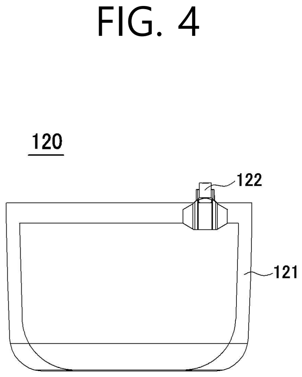

**FIG. 4** is a plan view of a drug reservoir illustrated in **FIG. 3****.** Referring to **FIG. 4****,** the drug reservoir 120 stores a drug to be injected, and its thickness varies depending on the amount of drug contained therein. The drug reservoir 120 may be provided as a flat pouch-type bag formed of a polymer material. A sealing portion 121 may be formed along the periphery of the drug reservoir 120 by bonding the edges of its two surfaces over a predetermined width. The sealing portion 121 may restrict expansion of the drug reservoir 120 toward its periphery.

The drug reservoir 120 may be formed of a polymer film or the like, and inner and outer layers of the drug reservoir 120 may be formed of materials having different melting points. Herein, the melting point of the inner layer is lower than that of the outer layer. The drug reservoir 120 is manufactured by applying heat at a predetermined temperature sufficient to melt the inner material to form the sealing portion 121. When heat is applied to a side surface of the sealing portion 121, the inner materials adhere to each other to form a seal. Also, when a lower portion is folded into a W-shape and heated, the inner materials adhere to each other, whereas the outer materials do not, which results in a corrugated seal. Unlike conventional techniques, the drug reservoir 120 is thinly formed of a flexible material. Thus, the patch-type drug injection device 100 can be implemented to be thinner and lighter.

Further, a drug inlet/outlet port 122 is coupled to the drug reservoir 120. Thus, a drug can flow into the drug reservoir 120 or a stored drug can be discharged to the outside.

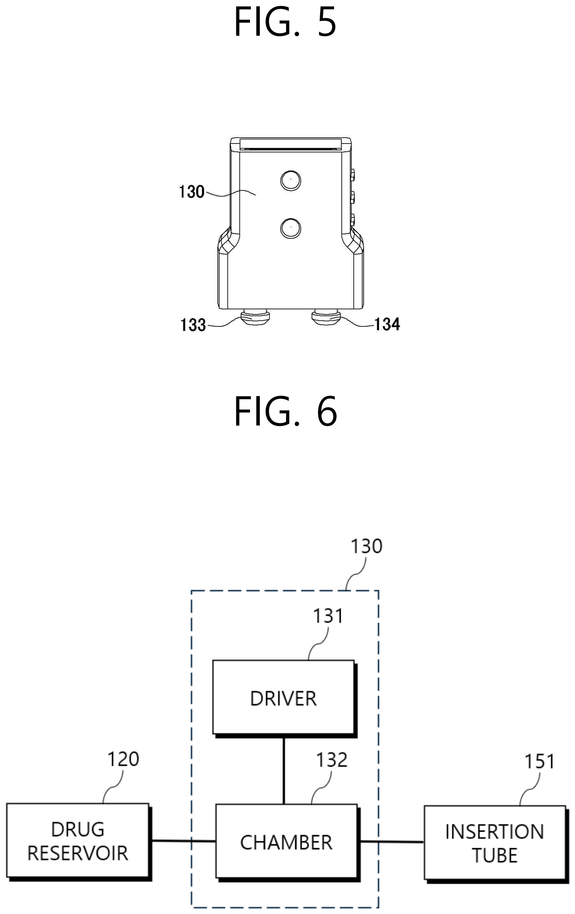

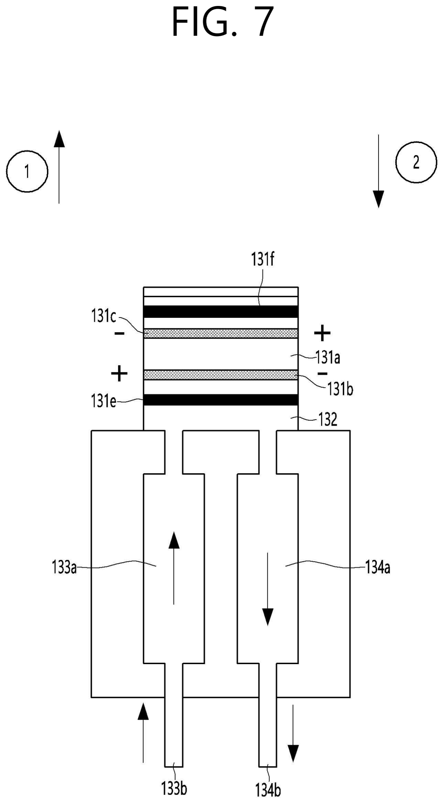

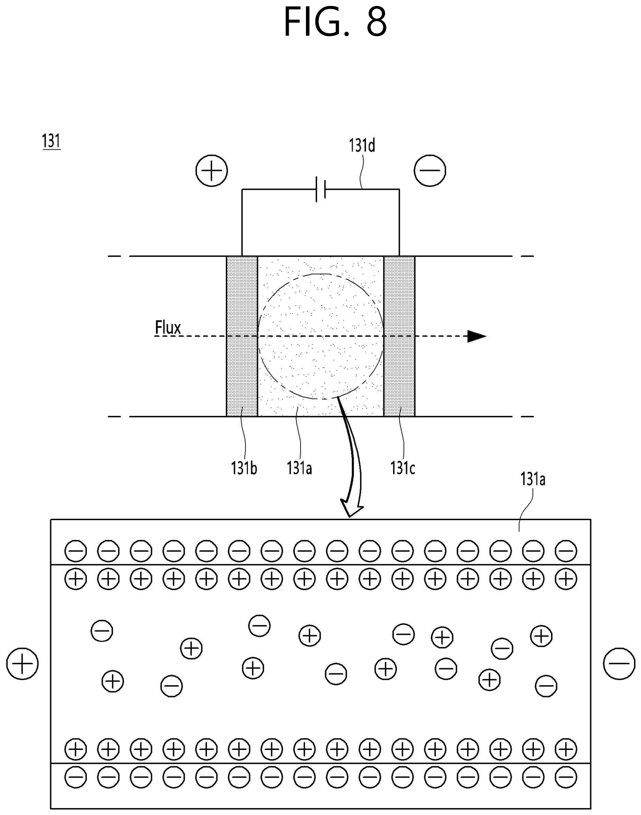

**FIG. 5** is a plan view of a pump assembly illustrated in **FIG. 3****,** **FIG. 6** is a block diagram schematically illustrating a configuration of the pump assembly illustrated in **FIG. 5****,** **FIG. 7** is a block diagram schematically illustrating a configuration of a driver illustrated in **FIG. 6****,** and **FIG. 8** is an example diagram schematically illustrating an operation of the driver illustrated in **FIG. 7****.**

The pump assembly 130 will be described in detail with reference to **FIG. 5** to **FIG. 8****.**

Referring to **FIG. 5** and **FIG. 6****,** the pump assembly 130 is an electrochemically driven pump, and includes a driver 131, a transfer chamber 132, a suction unit 133, and a discharge unit 134. The driver 131 is electrochemically driven in response to a control signal to generate positive and negative pressures. Thus, a drug is sucked from the drug reservoir 120 and then stored in the transfer chamber 132. The drug stored in the transfer chamber 132 is discharged to the injection assembly 150. The transfer chamber 132 receives and contains the drug sucked from the drug reservoir 120 by the driver 131. The suction unit 133 delivers the drug from the drug reservoir 120 to the transfer chamber 132, and the discharge unit 134 discharges the drug from the transfer chamber 132 to an insertion tube 151 of the injection assembly 150. Herein, the drug needs to be injected into a specific patient and may be, for example, insulin that is injected into a diabetic patient.

The driver 131 will be described in detail with reference to **FIG. 7** and **FIG. 8****.** The driver 131 is a pump that uses the movement of a fluid according to electroosmosis that occurs when a voltage or current is applied by using electrodes at both ends of a pressure generator (membrane) 131a. The driver 131 may include the membrane 131a, a power source 131d that applies a voltage or current to a first electrode 131b and a second electrode 131c arranged on both sides of the membrane 131a, and first and second diaphragms 131e and 131f through which a fluid moves. The first and second diaphragms 131e and 131f are respectively provided on one side and on the other side of the membrane 131a, and are deformed in shape by the movement of a pumping solution as positive and negative pressures are alternately generated. For example, the first diaphragm 131e and the second diaphragm 131f transfer the positive and negative pressures generated by an operation of the membrane 131a to a transfer target fluid. More specifically, when a negative pressure is generated, at least a part of the first diaphragm 131e and the second diaphragm 131f moves backwards (*i.e.,* moves in a direction ①) such that the transfer target fluid is sucked to the transfer chamber 132, and when a positive pressure is generated, at least a part of the first diaphragm 131e and the second diaphragm 131f moves forwards (i.e., moves in a direction ②) such that the transfer target fluid is discharged from the transfer chamber 132.

Silica, glass, and the like are generally used as a material of the membrane 131a, and when the silica and glass are immersed in an aqueous solution, surfaces thereof are negatively charged. There are many paths, through which fluids may pass, in the membrane 131a, and when one of the paths is enlarged, a surface of the fluid path with negative charges (bound anions) may be in a state in which the negative charges are balanced by mobile cations having positive charges. In this state, when a positive voltage is applied to the first electrode 131b and a negative voltage is applied to the second electrode 131c, a negative pressure is generated, and the fluid inside the driver 131 moves in the direction ① due to the negative pressure. In this case, the drug is sucked through an inflow path 133b and flows into the transfer chamber 132 through a first check valve 133a. In this case, a second check valve 134a is closed such that the negative pressure is not transferred to an outflow path 134b. When a negative voltage is applied to the first electrode 131b and a positive voltage is applied to the second electrode 131c, a positive pressure in the opposite direction is generated by a reversible electrochemical reaction. The fluid inside the driver 131 moves in the direction ② by the positive pressure. In this case, the drug stored in transfer chamber 132 is injected into a target through the second check valve 134a and the outflow path 134b. In this case, the first check valve 133a is closed such that the positive pressure is not transferred to the inflow path 133b.

This phenomenon is called electroosmosis, and a pump that uses this principle is an electroosmotic pump. Since the patch-type drug injection device 100 of the present disclosure uses the pump assembly 130 as a driving means, the overall thickness can be reduced, and the device can be more stably attached to the skin.

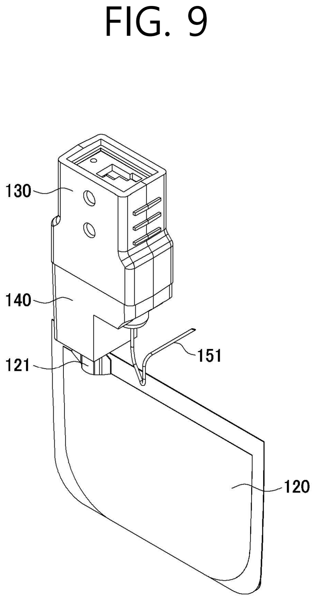

**FIG. 9** is a perspective view illustrating a coupling structure of the drug reservoir, the pump assembly, and the port connector illustrated in **FIG. 3****.**

Referring to **FIG. 9****,** the port connector 140 is disposed between the drug reservoir 120 and the pump assembly 130 and coupled to the drug reservoir 120 and the pump assembly 130. Also, the port connector 140 provides a flow path through which the drug sucked from the drug reservoir 120 to the pump assembly 130 moves and a flow path through which the drug discharged from the pump assembly to the insertion tube 151 of the injection assembly 150 moves.

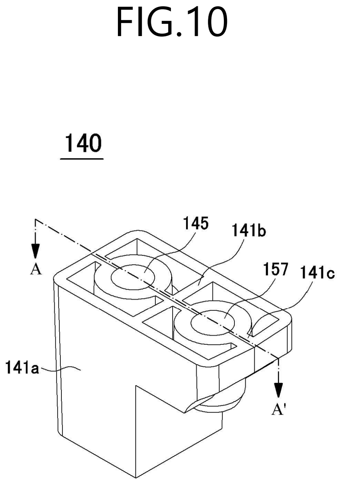

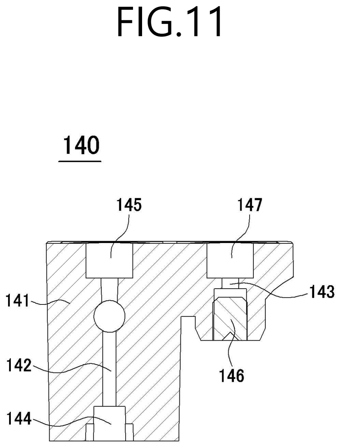

**FIG. 10** is a front perspective view of the port connector illustrated in **FIG. 3****,** and **FIG. 11** is a cross-sectional view taken along a line A-A' of **FIG. 10****.**

Referring to **FIG. 10** and **FIG. 11****,** the port connector 140 is coupled to the drug reservoir 120, the pump assembly 130, and the insertion tube 151 of the injection assembly 150, and includes a main body 141 in which a first flow path 142 and a second flow path 143 are formed.

The main body 141 may include a housing 141a, a partition wall 141b, and a support portion 141c. The housing 141a has a predetermined internal space, and the partition wall 141b is disposed within the housing 141a to divide the internal space of the housing 141a into a first space and a second space. The first flow path 142 is disposed in the first space and the second flow path 143 is disposed in the second space. An end of the housing 141a and an end of the partition wall 141b may be formed to have a predetermined area for hermetic coupling with an end of the pump assembly 130.

The support portion 141c connects the housing 141a to the first flow path 142 or the second flow path 143, or connects the partition wall 141b to the first flow path 142 or the second flow path 143. Thus, the support portion 141c can fix the first flow path 142 and the second flow path 143.

The first flow path 142 serves as a flow path through which a drug moves from the drug reservoir 120 to the pump assembly 130, and the second flow path 143 serves as a flow path through which a drug moves from the pump assembly 130 to the insertion tube 151.

Herein, the first flow path 142 and the second flow path 143 may be formed into a cylindrical shape, and the length of the first flow path 142 may be greater than that of the second flow path 143. The first flow path 142 and the second flow path 143 are formed parallel and adjacent to each other within the main body 141, and may have the shape illustrated in **FIG. 11** due to the difference in length between the first flow path 142 and the second flow path 143. In the drug injection device 100 of the present disclosure, the injection assembly 150 configured to inject a drug into the user may be disposed beneath the second flow path 143 of the port connector 140, as shown in **FIG. 4****,** according to the shape of the port connector 140. Thus, spatial utilization can be improved and the overall size of the device can be reduced. Therefore, it is possible to minimize user discomfort.

Further, one end of the first flow path 142 is connected to the drug reservoir 120 and the other end is connected to the pump assembly 130. Furthermore, one end of the second flow path 143 is connected to the injection assembly 150, and the other end is connected to the pump assembly 130. To achieve this connection structure, the main body 141 includes first to fourth coupling grooves 144, 145, 146 and 147. The first coupling groove 144 is formed on one side of the first flow path 142, and the drug inlet/outlet port 122 of the drug reservoir 120 is inserted and coupled to the first coupling groove 144. The second coupling groove 145 is formed on the other side of the first flow path 142, and the suction unit 133 of the pump assembly 130 is inserted and coupled to the second coupling groove 145. The third coupling groove 146 is formed on one side of the second flow path 143, and the insertion tube 151 of the injection assembly 150 is coupled to the third coupling groove 146. The fourth coupling groove 147 is formed on the other side of the second flow path 143, and the discharge unit 134 of the pump assembly 130 is inserted and coupled to the fourth coupling groove 147. Herein, the third coupling groove 146 may further include a sealing member (not shown) for sealing a coupling portion with the insertion tube 151.

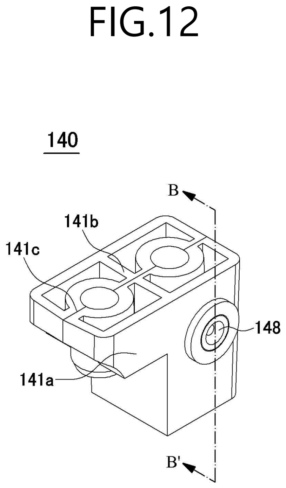

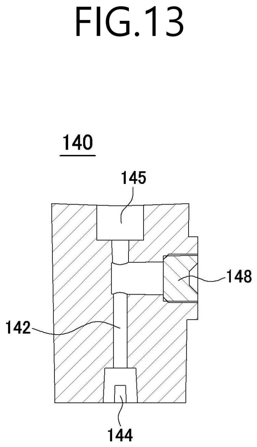

**FIG. 12** is a rear perspective view of the port connector, and **FIG. 13** is a cross-sectional view taken along a line B-B' of **FIG. 12****.**

Referring to **FIG. 12** and **FIG. 13****,** the port connector 140 may further include a drug injection path 148 formed in the main body 141. The drug injection path 148 is formed in a direction intersecting a longitudinal direction of the first flow path 142, one end of which communicates with the first flow path 142 and the other end of which is open to the outside. The drug injection path 148 serves as a flow path through which a drug can be injected into the first flow path 142 by means of a drug injection syringe, and is used to fill the drug reservoir 120 with a drug at the initial stage of operation of the drug injection device 100 of the present disclosure.

Thus, the port connector 140 serves as a medium for delivering a drug between the drug reservoir 120 and the pump assembly 130, and between the pump assembly 130 and the injection assembly 150. Therefore, it is possible to enhance the versatility of the pump assembly 130. When the pump assembly 130 is directly connected to the drug reservoir 120 and the injection assembly 150, the structure of the pump assembly 130 needs to be modified according to changes in the shape of a connection portion of the drug reservoir 120 or the injection assembly 150. However, by using the port connector 140, the structure of the pump assembly 130 can be maintained while only the connection portions of the port connector 140 are modified according to changes in the structures of the drug reservoir 120 and the injection assembly 150. Thus, it is possible to enhance the versatility of the pump assembly 130.

Referring back to **FIG. 4****,** the injection assembly 150 includes the insertion tube 151 to inject a drug discharged from the pump assembly 130 into an injection target. The circuit unit 160 is equipped with electronic components necessary for driving and controlling the pump assembly 130, and may include a battery 161 for supplying power.

Hereafter, referring to **FIG. 1** and **FIG. 2****,** additional components of the patch-type drug injection device 100 will be described.

Referring to **FIG. 1****,** the lid 111 of the housing 110 has a shooting hole 111a formed in a region corresponding to the injection assembly 150. The shooting hole 111a is a hole into which a shooting member of a shooting device (not shown) is inserted to press the injection assembly 150, thereby allowing the insertion tube 151 to be inserted into the injection target.

Referring to **FIG. 2****,** a drug injection hole 112b and an air vent hole 112c are formed in the contact surface 112 of the housing 110. The drug injection hole 112b is a hole for supplying a drug from the outside into the drug reservoir 120, and the air vent hole 112c is a hole for reducing a pressure difference between the inside and the outside of the housing 110. The drug injection hole 112b is formed at a position corresponding to the drug injection path 148 formed in the port connector 140. Thus, a drug can be injected through the drug injection hole 112b and the drug injection path 148 by means of a syringe or the like.

Further, the patch-type drug injection device 100 may include a sound output device (not shown) within the housing 110 to provide a notification of a specific condition. The circuit unit 160 includes a sound output circuit capable of stopping the operation of the sound output device (not shown), and a buzzer stop hole 112d may be formed in the contact surface 112 to provide a passage for operating the sound output circuit of the circuit unit 160. Herein, silicone caps may be coupled to the discharge port 112a and the drug injection hole 112b, and air vent stickers may be attached to the air vent hole 112c and the buzzer stop hole 112d.

It would be understood by a person with ordinary skill in the art that various changes and modifications may be made based on the above description without changing technical conception and essential features of the present disclosure. Thus, it is clear that the above-described embodiments are illustrative in all aspects and do not limit the present disclosure. The scope of the present disclosure is defined by the following claims. It shall be understood that all modifications and embodiments conceived from the meaning and scope of the claims and their equivalents are included in the scope of the present disclosure.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A port connector coupled to a pump assembly and a drug reservoir of a drug injection device, comprising:
a main body coupled between the drug reservoir and the pump assembly; and
first and second flow paths which are formed inside the main body and through which a drug moves,
wherein the first flow path is a flow path through which the drug moves from the drug reservoir toward the pump assembly, and
the second flow path is a flow path through which the drug discharged from the pump assembly moves.

2. The port connector of Claim 1,
wherein the first flow path and the second flow path are formed parallel to each other, and the second flow path has a shorter length than the first flow path.

3. The port connector of Claim 1,
wherein the main body includes:
a housing having a predetermined space; and
a partition wall disposed within the housing to divide the space into a first space and a second space, and
the first flow path is disposed in the first space and the second flow path is disposed in the second space.

4. The port connector of Claim 3,
wherein each of the first flow path and the second flow path is formed into a cylindrical shape, and
the main body further includes:
a support portion that connects the housing including the first space and the second space to the first flow path or the second flow path, or connects the partition wall to the first flow path or the second flow path.

5. The port connector of Claim 3,
wherein an end of the housing and an end of the partition wall are formed to have a predetermined area for hermetic coupling with an end of the pump assembly.

6. The port connector of Claim 1,
wherein one end of the first flow path is coupled to the drug reservoir and the other end is coupled to the pump assembly, and
one end of the second flow path is coupled to an insertion tube configured to deliver the drug to a user and the other end is coupled to the pump assembly.

7. The port connector of Claim 6,
wherein the main body includes:
a first coupling groove which is formed on one side of the first flow path and to which a drug inlet/outlet port of the drug reservoir is inserted and coupled; and
a second coupling groove which is formed on the other side of the first flow path and to which a suction unit of the pump assembly is inserted and coupled.

8. The port connector of Claim 6,
wherein the main body includes:
a third coupling groove which is formed on one side of the second flow path and to which the insertion tube is coupled; and
a fourth coupling groove which is formed on the other side of the second flow path and to which a discharge unit of the pump assembly is inserted and coupled, and
the third coupling groove further includes a sealing member for sealing a coupling portion with the insertion tube.

9. The port connector of Claim 1,
wherein the main body further includes:
a drug injection path formed in a direction intersecting a longitudinal direction of the first flow path.

10. The port connector of Claim 9,
wherein one side of the drug injection path is connected to the first flow path and the other side is open to the outside of the main body.

11. The port connector of Claim 10,
wherein the main body further includes:
a sealing member, into which a drug injection syringe is to be inserted, in the drug injection path.

12. A drug injection device, comprising:
a housing which includes a lid and a contact surface and in which a mounting space is formed between the lid and the contact surface;
a drug reservoir which is disposed in the mounting space and in which a drug is stored;
a pump assembly which electrochemically generates positive and negative pressures to suck and discharge the drug from the drug reservoir;
a port connector which is disposed between the drug reservoir and the pump assembly and coupled to the drug reservoir and the pump assembly, and provides a first flow path through which the drug sucked from the drug reservoir to the pump assembly moves and a second flow path through which the drug discharged from the pump assembly moves; and
an insertion tube coupled to the port connector and configured to inject the drug into a user.

13. The drug injection device of Claim 12,
wherein the port connector includes:
a main body which is coupled to the drug reservoir, the pump assembly, and the insertion tube and in which the first flow path and the second flow path are formed, and
the first flow path is a flow path through which the drug moves from the drug reservoir toward the pump assembly, and
the second flow path is a flow path through which the drug moves from the pump assembly to the injection assembly.

14. The drug injection device of Claim 13,
wherein the first flow path and the second flow path are formed parallel to each other, and
the second flow path has a shorter length than the first flow path.

15. The drug injection device of Claim 13,
wherein the main body includes:
a housing having a predetermined space; and
a partition wall disposed within the housing to divide the space into a first space and a second space, and
the first flow path is disposed in the first space and the second flow path is disposed in the second space.

16. The drug injection device of Claim 15,
wherein each of the first flow path and the second flow path is formed into a cylindrical shape, and
the main body further includes:
a support portion that connects the housing including the first space and the second space to the first flow path or the second flow path, or connects the partition wall to the first flow path or the second flow path.

17. The drug injection device of Claim 15,
wherein an end of the housing and an end of the partition wall are formed to have a predetermined area for hermetic coupling with an end of the pump assembly.

18. The drug injection device of Claim 12,
wherein the pump assembly includes:
a driver configured to electrochemically generate positive and negative pressures in an alternating manner;
a transfer chamber frame which is coupled to one side of the driver and in which a transfer chamber is formed to receive and contain the drug; and
a suction unit and a discharge unit which are coupled to one side of the transfer chamber frame and connected to the transfer chamber.

19. The drug injection device of Claim 18,
wherein the main body includes:
a first coupling groove which is formed on one side of the first flow path and to which a drug inlet/outlet port of the drug reservoir is inserted and coupled; and
a second coupling groove which is formed on the other side of the first flow path and to which the suction unit is inserted and coupled.

20. The drug injection device of Claim 19,
wherein the main body includes:
a third coupling groove which is formed on one side of the second flow path and to which the insertion tube of the injection assembly is coupled; and
a fourth coupling groove which is formed on the other side of the second flow path and to which the discharge unit is inserted and coupled, and
the third coupling groove further includes a sealing member for sealing a coupling portion with the insertion tube.

21. The drug injection device of Claim 13,
wherein the main body further includes:
a drug injection path formed in a direction intersecting a longitudinal direction of the first flow path.

22. The drug injection device of Claim 21,
wherein one end of the drug injection path is connected to the first flow path and the other end is open to the outside of the main body.

23. The drug injection device of Claim 22,
wherein the main body further includes:
a sealing member, into which a drug injection syringe is to be inserted, in the drug injection path.
